# EUROPEAN PATENT SPECIFICATION

(11) **EP 3 936 110 B1**
(45) Date of publication and mention of the grant of the patent: **31.08.2022**
(21) Application number: 20184514.6
(22) Date of filing: 07.07.2020
(51) Int. Cl.: A61K 8/49, A61Q 7/00

(54) **TREATMENT OF SEASONAL HAIR THINNING**
BEHANDLUNG VON SAISONALER HAARLICHTUNG
TRAITEMENT DE LA CHUTE DE CHEVEUX SAISONNIÈRE

(43) Date of publication of application: 12.01.2022
(73) Proprietor: Dr. Kurt Wolff GmbH & Co. KG, 33611 Bielefeld (DE)
(72) Inventor: Schulze zur Wiesche, Erik, 33611 Bielefeld (DE); Becker, Maike, 33611 Bielefeld (DE); Völker, Jörn Michael, 33611 Bielefeld (DE); Koch, Nadine, 33611 Bielefeld (DE)
(74) Representative: Ter Meer Steinmeister & Partner

(56) References cited:
- WO-A1-2013/098333
- DE-A1-102005 003 949
- US-A1- 2009 264 449
- US-A1- 2012 157 478
- E.Y. HSIANG ET AL: "Seasonality of hair loss: a time series analysis of Google Trends data 2004-2016", BRITISH JOURNAL OF DERMATOLOGY, vol. 178, no. 4, 7 February 2018 (2018-02-07), pages 978-979, XP055751933, UK ISSN: 0007-0963, DOI: 10.1111/bjd.16075
- MICHAEL KUNZ ET AL: "Seasonality of Hair Shedding in Healthy Women Complaining of Hair Loss", DERMATOLOGY, vol. 219, no. 2, 1 January 2009 (2009-01-01), pages 105-110, XP055751878, CH ISSN: 1018-8665, DOI: 10.1159/000216832
- DEEPA LIYANAGE ET AL: "Telogen Effluvium", COSMETICS, vol. 3, no. 2, 25 March 2016 (2016-03-25), page 13, XP055751998, DOI: 10.3390/cosmetics3020013
- M.G. DAVIS ET AL: "A novel cosmetic approach to treat thinning hair", BRITISH JOURNAL OF DERMATOLOGY, vol. 165, 16 August 2011 (2011-08-16), pages 24-30, XP055752004, UK ISSN: 0007-0963, DOI: 10.1111/j.1365-2133.2011.10633.x

## Description

The present invention provides methods for preventing and/or treating seasonal hair thinning in a mammal. Moreover, the present invention relates to cosmetic and pharmaceutical compositions containing caffeine and cosmetically and pharmaceutically acceptable excipients for use in preventing and/or treating seasonal hair thinning.

Fluctuations in hair growth rates and hair diameter of the human scalp hair during the seasonal changes of the year have been described (Randall et al, Seasonal changes in human hair growth, Br J Dermatol. 1991 Feb, 124(2):146-51 and Liu et al, Changes in Chinese hair growth along a full year, Int J Cosm Sci 2014, 36(6):531-536).

In particular, beard hair growth showed significant seasonal variations, with lowest growth rates in winter (January till March) and higher growth rates in end of summer (June till August) (Randall et al, Seasonal changes in human hair growth, Br J Dermatol. 1991 Feb, 124(2):146-51). Observations back in 1937 also showed that higher temperature in summer corresponds to higher growth rates of beard hair in comparison to lower temperatures and lower growth rates in winter (Eaton et al, Temperature and the growth of the hair. Science1937, 86(2233):354). Further, seasonal hair growth rates in the adult domestic cat showed a sinusoidal pattern throughout the year, similar to that found for day length and daily mean air temperature, with a maximum hair growth rate in summer and a minimum hair growth rate in winter (Hendriks et al, Seasonal hair growth in the adult domestic cat (felis catus), Comp. Biochem. Physiol., A: Physiol. 1997. 116A(1):39-35).

The relevance of human scalp hair goes far beyond its physiological protective role. The appearance and feel of human scalp hair indicates culture, health, youth, lifestyle and attractiveness. Human scalp hair is an outstanding tool for non-verbal communication, and therefore, an important interpersonal factor. Beyond that healthy hair has a high priority for both men and woman. Healthy scalp hair is a hair with its full capacity of growing healthy in both diameter and length. In contrast, hair thinning and hair loss has a great negative influence not only on quality of life, including the lack of self-esteem, self-image and selfconfidence as well as more severely the development of depression and anxiety. Several studies have even shown that the psychological burden of hair loss is even more severe and devastating for women compared to the impact of hair loss in men (Davis et al, Review of quality of life studies in women with alopecia, Int. Journal of Women's Dermatology 4 (2018) 18-22). Additionally, since thin hair is much weaker and more prone to breakage, people suffering from seasonal hair thinning are more severely facing problems during the daily grooming practices. Women suffering from thin hair have to pay much more attention on their daily hair care, during washing, drying combing etc. to avoid hair breakage.

There is a wide selection of scalp hair care products, such as shampoos, conditioners, hair sprays, mousses and gels to influence the physical and mechanical properties of the single hair fiber. The scalp hair care products normally utilize conditioning substances such as polyquaternium derivatives, silicones and/or protein hydrolysates. These substances are suitable to build up the surface of the keratin fiber which results in an improved feel and appearance (e.g. softness and shine) of the scalp hair.

However, since these substances are built up on the outer surface of the hair and give each hair fiber more weight, women suffering from thin hair are consequently facing the burden of flat hair that lacks of volume. Finally, the scalp becomes more visible, which is an undesired situation for women in general and for women with thin hair in particular.

WO 2013/098333 relates to the combination of cytokines, growth factors, chemotactic factors, stem cell stimulating factors, antibacterial/antiviral factors, complement C3a/C4a proteins and immunoglobulins, each in specific amounts, for use in treating conditions requiring tissue repair and regeneration and for the substitution of stem cell therapies/transplants. DE 10 2005 003949 relates to the use of caffeine in the prevention of androgenetic alopecia. US 2009/264449 and US 2012/157478 disclose hair care compositions comprising inter alia a xanthine compound (such as caffeine), niacinamide and panthenol. Liyanage et al., Cosmetics, 2016, 3, 13 focus on "thinning hair" in connection with female pattern hair loss and telogen effluvium.

Hence, there is a strong demand for an effective, safe and sustainable treatment and/or prevention of seasonal hair thinning.

In view of the unresolved deficiencies in treating seasonal hair thinning as discussed above, it is an object of the present invention to provide an effective, safe and sustainable treatment and/or prevention of seasonal hair thinning.

The above-mentioned object has surprisingly been solved by the inventors of the present invention in that they found out that caffeine, when applied topically, significantly and sustainably decreases or reduces hair thickness loss (i.e. reduce hair thinning) leading to thicker growing hair. It was particularly surprising that these effects are significant for so-called "winter hair", namely seasonally thinned hair grown from January to April, while there was no significant difference for so-called "summer hair", namely hair grown from July to October. Without wishing to be bound by theory, the inventors believe that the topical application of caffeine onto the scalp (i.e. to the root of the hair) effectively stimulate hair root activities leading to a significant hair thickness growth of winter hair. In other words, according to the invention actually thicker growing winter hair is obtained rather than only a temporary swelling of the keratin fibers, the latter apparently being caused by substance deposition on or in the cuticle of the single keratin fibers. Thus, according to the invention a long-term modification of the actual dimensional properties of the single hair keratin fiber is provided presumably by supporting the biological relevant mechanisms to reduce hair thinning in a sustainable manner.

The term of "seasonal hair thinning" as used according to the present invention is defined as the reduction of hair diameter during the winter season (i.e. winter hair, such as from January to April having average temperatures below 10°C) as compared to the summer season (i.e. summer hair, such as from July to October having average temperatures above 11°C). In other words, "seasonal hair thinning" herein is also understood as the difference in the average diameter between winter hair and summer hair and preferably means a seasonal phenomenon, which leads to the reduction of hair diameter by up to 10%, as determined by optical fibre diameter analyzer/OFDA. Alternative terms having the same meaning are winter hair thinning, cold temperature hair thinning, seasonal reduction of hair diameter, seasonal hair fiber weakening, seasonal hair weakening or seasonal hair diameter atrophy.

In particular, the inventors of the present invention surprisingly found out that the topical application of caffeine is able to overcome or at least to significantly limit the seasonal hair thinning. In other words, it was surprisingly found that the topical application of caffeine significantly increases the hair diameter of winter hair. In this way the present invention overcomes the seasonal differences in hair thicknesses and prevents and/or treats the seasonal hair thinning accordingly. Effective prevention and/or treatment is understood herein as the increase of the average diameter of winter hair by at least 1%, in particular by 1 to 10%, as determined by optical fibre diameter analyzer/OFDA.

Therefore, the subject-matter of the present invention is the cosmetic (non-therapeutic) use of a composition comprising caffeine for preventing and/or treating seasonal hair thinning.

The following definitions are relevant in connection with the embodiments of the present invention.

The meaning of the term "comprising" is to be interpreted as encompassing all the specifically mentioned features as well optional, additional, unspecified ones, whereas the term "consisting of' only includes those features as specified. Therefore, "comprising" includes as a limiting case the meaning of "consisting of'.

The term "% by weight" or "weight-%" or "wt.-%" is to be understood to refer to the weight amount of the component relative to the total weight amount of the respective composition, if not specified otherwise.

Preferred embodiments according to the invention are defined hereinafter. The preferred embodiments are preferred alone or in combination. Further, it is to be understood that the following preferred embodiments refer to all aspects of the present invention. Preferred embodiment herein means that the effect achieved according to the invention, namely to limit the seasonal hair thinning, is particularly significant.

According to the invention caffeine is present in the composition in a quantity of 0.1% to 10% by weight, preferably in a quantity of 0.1% to 5% by weight. A particularly preferred range is from 0.2% to 2% by weight. Most preferred values are 0.1%, 0.2%, 0.3%, 0.4%, 0.5%, 0.6%, 0.7%, 0.8%, 0.9% and 1.0%, each by weight.

When using these quantities of caffeine the seasonal hair thinning is most effectively prevented and/or treated. As mentioned above, effective prevention and/or treatment is understood herein as the increase of the average diameter of winter hair by at least 1%, in particular by 1 to 10%, such as by 2, 3, 4, 5, 6, 7, or 8%, as determined by optical fibre diameter analyzer/OFDA. In other words, based on the value of up to 10% hair diameter reduction during the natural phenomenon of "seasonal hair thinning" as mentioned above, according to the present invention the seasonal hair thinning is reduced by 50 to 100%, preferably by 60 to 90%, and particularly preferably by at least 65% and/or up to 80% (in particular by 67%, 70%, 75% or 80%).

In a preferred embodiment of the invention the composition further comprises at least one additional active ingredient being at least one compound selected from niacin, niacinamide, zinc PCA, zinc chloride, panthenol, allantoin, bisabolol, biotin, salicylic acid, piroctone olamine, fumaric acid, zinc pyrithione, glycerin, hydrolyzed wheat protein, triticum vulgare gluten, menthol, soy extract, glycine soy oil, white tea extract, phytosterols, persea gratissima oil, olea europaea fruit oil, tocopherol, tocopheryl acetate, taurin, calcium gluconate, and/or magnesium gluconate. Preferably, the composition comprises a mixture of one or more of these additional components.

In a preferred embodiment of the invention the composition further comprises at least one additional component selected from the group consisting of hair conditioning agents, skin conditioning agents, moisturizing agents, preservatives, viscosifiers, stabilizers, pH-modifiers, fragrances, antioxidants, surfactants, light protection filters, colouring dyes, chelators, and solvents. In addition, vitamins, flavonoids, phytoflavones, light protection filters, desquamation actives, anti-atrophy actives, chelators, terpenoids and terpenes, saccharides, plant extracts, oligopeptides, polypeptides, and other secondary plant accompanying substances may be present.

In a preferred embodiment, the composition comprises a preservative independently selected from the group consisting of benzoic acid, benzyl alcohol, chlorocresol, paraben esters, sorbic acid, ascorbic acid and salts thereof, sodium metabisulfite, chelating agents, citric acid, salicylic acid, and mixtures thereof.

Moreover, in a preferred embodiment, the composition comprises a pH modifier independently selected from the group consisting of diethanolamine, lactic acid, monoethanolamine, sodium hydroxide, sodium phosphate, triethanolamine, and mixtures thereof.

In a preferred embodiment, the composition comprises a surfactant selected from the group consisting of non-ionic surfactants, anionic surfactants, cationic surfactants, amphoteric surfactants and mixtures thereof.

Surfactants are molecules that comprise a hydrophilic and a hydrophobic moiety at opposite ends. Suitable surfactants are described in "Cosmetic Formulation of Skin Care Products", Draelos et al., Taylor & Francis Group, 2006.

In an embodiment, the non-ionic surfactant is selected from the group consisting of C₄-C₂₆ fatty alcohol ethoxylates, alkylphenol ethoxylates, ethoxylated amines/amides, glycerol C₄-C₂₆ fatty acid amides, glycerol C₄-C₂₆ fatty acid esters, sorbitol fatty acid esters, and mixtures thereof. The prefix "Cₓ-C_{y}" denotes the number of carbon atoms of the respective functional group. Non-limiting examples of a C₄-C₂₆ fatty alcohol ethoxylates are pentaethylene glycol monododecyl ether (also referred to as C12E5), hexaethylene glycol monododecyl ether (C12E6), heptaethylene glycol monododecyl ether (C12E7), and octaethylene glycol monodecyl ether (C12E8). Non-limiting examples of alkylphenol ethoxylates are Triton X-100^{®} (octyl phenol ethoxylate) and Nonidet P-40^{®} (4-nonylphenol ethoxylate). Non-limiting examples of ethoxylated amines/amides are polyethoxylated tallow amine, cocamide monoethanolamine, and cocamide diethanolamine. Non-limiting examples of glycerol C₄-C₂₆ fatty acid esters are glycerol monostearate and glycerol monolaurate. Non-limiting examples of sorbitol fatty acid esters are sorbitan monolaurate, sorbitan monostearate and sorbitan tristearate.

Anionic surfactants dissociate in water to yield an anionic surfactant in water. Anionic surfactants useful herein include alkyl sulfates and alkyl ether sulfates. These materials have the respective formulae ROSO₃M and RO(C₂H₄O)ₓSO₃M wherein R is a C₄-C₂₆ alkyl or C₄-C₂₆ alkenyl, x is 1 to 10, and M is a water-soluble cation such as ammonium, sodium, potassium and triethanolamine. The alkyl ether sulfates useful in the present invention are condensation products of ethylene oxide and monohydric alcohols having about 4 to about 26 carbon atoms. Preferably, R is C₁₄-C₁₆ alkyl or C₁₄-C₁₈ alkenyl in both the alkyl and alkyl ether sulfates. The alcohols can be derived from fats, e.g., coconut oil or tallow, or can be synthetic. Lauryl alcohol and straight chain alcohols derived from coconut oil are preferred herein. Such alcohols are reacted with 1 to 10, and especially 3, molar proportions of ethylene oxide and the resulting mixture of molecular species, having, for example, an average of 3 moles of ethylene oxide per mole of alcohol, is sulfated and neutralized. Specific examples of alkyl ether sulfates of the present invention are sodium coconut alkyl trioxyethylene sulfate; lithium tallow alkyl trioxyethylene sulfate; sodium tallow alkyl hexaoxyethylene sulfate; and sodium lauryl ether sulfate. Additional examples of anionic surfactants which come within the terms of the present invention are the reaction product of fatty acids esterified with isethionic acid and neutralized with sodium hydroxide where, for example, the fatty acids are derived from coconut oil; sodium or potassium salts of fatty acid amides of methyl tauride in which the fatty acids, for example, are derived from coconut oil.

Cationic surfactants comprise primary, secondary and tertiary amines and quaternary ammonium salts, such as cetrimonium bromide, cetlypyridinium chloride, benzalkonium chloride, benzethonium chloride, dimethyldioctadecylammonium chloride and dioctadecyldimethylammonium bromide.

Amphoteric surfactants have an anionic and a cationic functional group. Examples of amphoteric surfactants are derivatives of aliphatic secondary and tertiary amines in which the aliphatic radical can be straight chain or branched and wherein one of the aliphatic substituents contains from about 8 to about 18 carbon atoms and one contains an anionic water solubilizing group, e.g., carboxy, sulfonate, sulfate, phosphate, or phosphonate. Specific examples of amphoteric surfactants are sodium 3-dodecylaminopropionate, sodium 3-dodecylaminopropane sulfonate, and *N*-alkyltaurines such as the one prepared by reacting dodecylamine with sodium isethionate. Particularly preferred amphoteric surfactants are betaines such as, for example, coco-amidopropyl betaines. The amphoteric surfactant component may be present in the composition in an amount ranging from about 5 to about 15% by weight, and preferably from about 9 to about 12% by weight, based on the weight of the composition.

In a particularly preferred embodiment, the composition comprises 1 to 4 vol.-% of a non-ionic surfactant.

In another preferred embodiment, the composition comprises a diluent independently selected from the group consisting of water and an organic solvent and mixtures thereof. Preferred organic solvents are glycerin, a glycol, sorbitol, ethanol, triacetin and mixtures thereof.

According to the present invention, the composition preferably comprises a further ingredient independently selected from the group consisting of vitamins, flavonoids, terpenes, saccharides, plant extracts, and mixtures thereof.

Non-limiting examples of vitamins are niacin, biotin, vitamin E (also referred to as tocopherol), vitamin A, and vitamin C. Vitamins are antioxidants and can have various additional useful properties.

Non-limiting examples of flavonoids are apigenin, luteolin, tangeritin, quercetin, kaempferol, myricetin, fisetin, galangin, isorhamnetin, pachypodol, rhamnazin, pyranoflavonols, and furanoflavonols.

Non-limiting examples of terpenes are farnesol, menthol, limonene, squalene, camphor, pinene, eucalyptol, retinol, geraniol, linalool, carvone and thymol.

Non-limiting examples of saccharides are saccharides derived from starch, sorbitol and maltitol.

Plant-extracts can have multiple purposes and are i.a. useful as natural antioxidant, antiaging, emollient, anti-inflammatory, anti-irritant, and/or antimicrobial component. Non-limiting examples of plant extracts are plant oils, such as soy oil, sunflower oil, almond oil, grape oil, avocado oil, castor oil, glycine soja oil, jojoba oil and oils from *Areca catechu, Crocus sativus, Curcuma longa,* green tea, *Crataevea murula, Rosmarinus Officinalis,* buckwheat seeds, *Emblica officinale, Ginkgo biloba, Centella asiatica, Psoraliea corylifolia, Citrus limonum, Aloe Vera, Matricaria recutita, Thea viridis, Vitis vinifera, Daucus carota, Lycopersicon esculentum, Allium sativum and Hamamelis virginiana.*

In another preferred embodiment, the composition comprises niacinamide. In still another preferred embodiment, the composition comprises biotin.

In a preferred embodiment, the composition comprises hydrolyzed wheat protein. In yet another embodiment, the composition comprises an amino acid, preferably arginine or leucine.

According to the present invention the additional active ingredients and/or additional components as described herein are each present in the composition in a quantity of 0.001 % to 50% by weight, preferably in a quantity of 0.001% to about 20% by weight, more preferably in a quantity of 0.001% to about 10% by weight, even more preferably in a quantity of 0.01 to 2 wt.-%, based on the total weight of the composition.

According to the invention the composition is applied topically. Preferably the composition is (directly) applied onto the scalp (i.e. penetrating to the root of the hair) rather than only deposited on or in the cuticle of the single keratin fibers. In this way the composition according to the invention effectively stimulates hair root activities.

Preferably, the composition is present in the form of a shampoo, conditioner, spray, mousse, gel, wax, emulsion, lotion, tonic or liquid.

If the composition is left in contact with the scalp for an extended time period, it is also referred to as a leave-on composition (such as a tonic or liquid). If the composition is rinsedoff after application it is referred to as a rinse-off composition, being typically rinsed off after 1 to 10 minutes (such as a conditioner or shampoo).

According to the present invention, the composition preferably comprises a surfactant and 0.1 to 10 wt.-% of caffeine. It is preferred that the composition is a shampoo. It is more preferred, that the composition is a caffeine shampoo comprising 0.1 to 5, or 0.2 to 1 wt.-% caffeine.

According to the present invention, the composition preferably comprises an organic solvent (preferably ethanol) and 0.1 to 10 wt.-% of caffeine. It is preferred that the composition is a tonic (namely a leave-on tonic). It is more preferred, that the composition is a caffeine tonic comprising 0.1 to 5, or 0.2 to 1 wt.-% caffeine.

The following compositions are preferred embodiments of the present invention:
In a typical embodiment the composition is a shampoo and contains 4.0 wt.-% sodium lauryl sulfate, 5.0 wt.-% sodium laureth sulfate, 1.2 wt.-% sodium lauroyl glutamate, 1.0 wt.-% hydroxypropyltrimonium hydrolyzed wheat, 3.0 wt.-% disodium laureth sulfosuccinate, 0.3 wt.-% biotin, 0.2 wt.-% tocopherylacetate, 0.2 wt.-% citric acid, 1 wt.-% caffeine, 0.3 wt.% fragrance, 0.2 wt.-% potassium sorbate, 0.5 % salicylic acid, 0.05 wt.-% niacinamide, and purified water.

In another typical embodiment the composition is a leave-on tonic and contains 40.0 wt.-% ethanol, 0.05 wt.-% tocopheryl acetate, 0.3 wt.-% PEG-40 hydrogenated castor oil, 0.3 wt.% caffeine, 0.2 wt.-% glycine soya oil, 0.3 wt.-% panthenol, 0.2 wt.-% fragrance, 0.1 wt.-% menthol, 0.05 wt.-% niacinamide, and purified water.

The following examples describe the present invention in more detail.

### Examples:

### Example 1:

The following scalp tonic is provided.

| | |
|---|---|
| Alcohol denat. | 30.0% |
| Amodimethicone | 0.5% |
| Panthenol | 0.5% |
| Caffeine | 0.5% |
| Bisabolol | 0.2% |
| PEG-25 PABA | 0.5% |
| PEG-16 Hydrogenated Castor Oil | 0.5% |
| Parfum | 0.3% |
| Menthol | 0.3% |
| Lactic acid | 0.1% |
| Aqua | ad 100 |

### Example 2:

The following scalp tonic is provided.

| | |
|---|---|
| Alcohol denat. | 25.0% |
| Caffeine | 0.2% |
| Glycerin | 1.0% |
| Panthenol | 0.7% |
| Parfum | 0.3% |
| PEG-40 Hydrogenated Castor Oil | 0.5% |
| Citric acid | 0.1% |
| Aqua | ad 100 |

### Example 3:

The following shampoo is provided.

| | |
|---|---|
| Sodium Myreth Sulfate | 4.0% |
| Sodium Laureth Sulfate | 4.0% |
| Disodium Laureth Sulfosuccinate | 3.0% |
| Tocopheryl acetate | 0.3% |
| Citric Acid | 0.2% |
| Caffeine | 0.5% |
| Parfum | 0.3% |
| Potassium Sorbate | 0.2% |
| Sodium Benzoate | 0.1% |
| Aqua | ad 100 |

### Example 4:

The following shampoo is provided.

| | |
|---|---|
| Sodium Myreth Sulfate | 4.0% |
| Sodium Laureth Sulfate | 4.0% |
| Disodium Laureth Sulfosuccinate | 3.0% |
| Tocopheryl acetate | 0.3% |
| Citric Acid | 0.2% |
| Caffeine | 1.0% |
| Parfum | 0.3% |
| Potassium Sorbate | 0.2% |
| Sodium Benzoate | 0.1% |
| Aqua | ad 100 |

### Example 5:

The following conditioner is provided.

| | |
|---|---|
| Sodium Laureth-11 carboxylate | 1.0% |
| Polyquaternium-4/ Hydroxypropyl Starch Copolymer | 2.0% |
| Steareth-8 | 5.0% |
| Cetyl Alcohol | 2.0% |
| Parfum | 0.5% |
| Caffeine | 0.4% |
| Hydrolyzed Keratin | 0.5% |
| Citric Acid | 0.2% |
| Sodium Benzoate | 0.05% |
| Potassium Sorbate | 0.15% |
| Aqua | ad 100 |

### Example 6:

The following emulsion is provided.

| | |
|---|---|
| PEG-40 (castor oil) | 6.0% |
| Cetearyl alcohol | 0.3% |
| Cera Alba | 0.7% |
| Myristylmyristat | 1.0% |
| Hexyldecanol | 5.0% |
| Dicaprylylether | 4.0% |
| Dioctylcyclohexan | 3.0% |
| Vitis Vinifera | 0.5% |
| Tocopherylacetat | 1.0% |
| Octylmethoxycinnamat | 2.0% |
| 4-Isobutyl-Dibenzoylmethan | 1.0% |
| Ascorbylpalmitat | 0.2% |
| Retinylpalmitat | 0.05% |
| 1,4 Butylenglycol | 4.0% |
| Carbomer | 0.3% |
| Hexyldecanol (and) Hexyldecyllaurat | 1.0% |
| KOH | 0.6% |
| Caffeine | 0.5% |
| Parfum | 0.2% |
| Aqua | ad 100 |

### Comparative Example 1:

The following scalp tonic is provided. It basically corresponds to the scalp tonic of Example 1 but does not contain caffeine.

| | |
|---|---|
| Alcohol denat. | 30.0% |
| Amodimethicone | 0.5% |
| Panthenol | 0.5% |
| Bisabolol | 0.2% |
| PEG-25 PABA | 0.5% |
| PEG-16 Hydrogenated Castor Oil | 0.5% |
| Parfum | 0.3% |
| Menthol | 0.3% |
| Lactic acid | 0.1% |
| Aqua | ad 100 |

### Comparative Example 2:

The following scalp tonic is provided. It basically corresponds to the scalp tonic of Example 2 but does not contain caffeine.

| | |
|---|---|
| Alcohol denat. | 25.0% |
| Glycerin | 1.0% |
| Panthenol | 0.7% |
| Parfum | 0.3% |
| PEG-40 Hydrogenated Castor Oil | 0.5% |
| Citric acid | 0.1% |
| Aqua | ad 100 |

### Study design

A composition comprising caffeine (Example 2) demonstrated a clinically significant effect on the seasonal hair thinning versus a placebo without caffeine (Comparative Example 2).

A 6 months randomized, placebo-controlled and double-blinded clinical study was carried out in 67 healthy women (ages 18-40) who perceived themselves as having thinning hair.

At the first measurement point in time (t0: calendar week 44 and 45 corresponding to beginning of November) a hair strand of about 100 hairs was cut off directly from the scalp on the upper back of the head (vertex region).

The test product (verum= Example 2 or placebo = Comparative Example 2) was then used by the subjects themselves according to the instructions by the study personnel over a period of six months. The product (4-7 mL) had to be gently massaged over the entire scalp and the product had to be applied once daily using an amount of product, which corresponds to usual practice. At the second measuring point in time after 6 months of daily product treatments (t1 calender week 17 and 18 corresponding to end of April beginning of May) the grown hair strand of the same test site as at t0 was cut off directly from the scalp of the test site.

### Sample preparation

For the hair diameter measurements the area close to the hair root (3-4 cm), corresponding to the hair that was grown 3-4 months before the whole hair tress was cut from the scalp, was cut in 2 mm fibre sections by guillotine and evenly distributed on a slide. Samples from probands were pooled into 4 groups corresponding to verum before and after treatment as well as placebo before and after treatment.

### Hair diameter measurement via Optical Fibre Diameter Analyzer (OFDA)

Hair diameter measurements was conducted based on the IWTO specification IWTO 47-13 "Measurement of the mean distribution of fibre diameter of wool using an Optical Fibre Diameter Analyzer (OFDA)".

In brief:
Hair fibre snippets are cut from each group and were evenly spread onto a 70 mm x 70 mm glass slide. The fibre density on the slide was as prescribed between 15% and 25%. The slide was placed into the OFDA100 instrument and several images were taken, covering an area of about 1 mm to 1.5 mm. During the image capture an ultra-bright light-emitting diode (LED) light passes through the slide and optical system to form a specific image on the camera sensor. The light image is finally converted to an array of pixels, which have different greyscale values. The diameter of each fibre is estimated to a resolution of one-quarter pixel (about 1 µm) by grey-scale interpolation. Each fibre measurement is assigned to a diameter bin of 1 µm width. After correction for fibre orientation, the contents of the bins are analysed to calculate the mean fibre diameter and distribution of diameter within each sample group.

### Results (Placebo according to Comparative Example 2 and Verum according to Example 2)

| | **Placebo** | **Verum** | **Placebo vs. Verum** |
|---|---|---|---|
| **Timepoint** | t0 (grown hair from July - October) | t0 (grown hair from July - October) | **t-Test** |
| **Mean [µm]** | 67,45 | 67,2 | **difference: 0,25 not significant** |
| **Standard deviation [µm]** | 18,3 | 18,5 | |
| **n** | 8432 | 8517 | |
| **Timepoint** | t1 (grown hair from January - April) | t1 (grown hair from January - April) | |
| **Mean [µm]** | 62,9 | 65,7 | **difference: -2,8 significant** |
| **Standard deviation [µm]** | 19,6 | 19,1 | |
| **n** | 6472 | 8054 | |
| **Mean t-Test** | **t0 vs. t1 difference: -4,55 significant** | **t0 vs. t1 difference: -1,5 significant** | |

### Mean seasonal temperature at study site (Hamburg)

| | month | Mean temperature |
|---|---|---|
| Before treatment | July | 20,0 |
| | August | 19,4 |
| | September | 15,2 |
| | October | 11,4 |
| | | |
| After treatment | January | 2,2 |
| | February | 5,3 |
| | March | 7,0 |
| | April | 9,9 |

It becomes evident from the above that according to the present invention the topical application of caffeine surprisingly is able to limit the hair thinning during the winter season, leading to normal a hair diameter, whereas the corresponding placebo formulation without caffeine did not counteract hair thinning during the cold winter season.

## Claims

1. Cosmetic use of a composition comprising caffeine for preventing and/or treating seasonal hair thinning, wherein caffeine is present in the composition in a quantity of 0.1% to 10% by weight, and wherein seasonal hair thinning is defined as the reduction of hair diameter during the winter season as compared to the summer season.

2. Use according to claim 1, wherein caffeine is present in the composition in a quantity of 0.1% to 5% by weight.

3. Use according to claim 1 and/or claim 2, wherein the composition is applied topically.

4. Use according to any of claims 1 to 3, wherein the composition is present in the form of a cosmetically acceptable formulation comprising a shampoo, leave-on and rinse-off emulsion and gel-based conditioners, waxes and further ethanol- as well as waterbased tonics.

5. Use according to any of claims 1 to 4, wherein the composition further comprises at least one additional active ingredient selected from the group consisting of niacin, niacinamide, zinc PCA, zinc chloride, panthenol, allantoin, bisabolol, biotin, tocopherol, tocopheryl acetate, and taurin.

6. Use according to any of claims 1 to 5, wherein the composition further comprises at least one additional component selected from the group consisting of hair conditioning agents, skin conditioning agents, moisturizing agents, preservatives, viscosifiers, stabilizers, pH-modifiers, fragrances, antioxidants, surfactants, light protection filters, colouring dyes, chelators, and solvents.

7. Use according to claim 5 and/or claim 6, wherein each of the additional active ingredient and/or each of the additional component is present in the composition in a quantity of 0.001% to 50% by weight, preferably in a quantity of 0.001% to 20% by weight, more preferably in a quantity of 0.001% to 10% by weight.

## Patentansprüche

1. Kosmetische Verwendung einer Zusammensetzung, die Koffein enthält, zur Vorbeugung und/oder Behandlung von saisonaler Haarverdünnung, wobei das Koffein in der Zusammensetzung in einer Menge von 0,1 bis 10 Gew.-% vorhanden ist, und wobei die saisonale Haarverdünnung definiert ist als die Verringerung des Haardurchmessers während der Wintersaison im Vergleich zur Sommersaison.

2. Verwendung nach Anspruch 1, wobei das Koffein in der Zusammensetzung in einer Menge von 0,1 bis 5 Gew.-% vorhanden ist.

3. Verwendung nach Anspruch 1 und/oder Anspruch 2, wobei die Zusammensetzung topisch angewendet wird.

4. Verwendung nach einem der Ansprüche 1 bis 3, wobei die Zusammensetzung in Form einer kosmetisch verträglichen Formulierung vorliegt, die ein Shampoo, eine Leave-on- und Rinse-off-Emulsion und Conditioner auf Gelbasis, Wachse und ferner Ethanol- sowie Wasser-basierte Tonika umfasst.

5. Verwendung nach einem der Ansprüche 1 bis 4, wobei die Zusammensetzung weiterhin mindestens einen zusätzlichen Wirkstoff umfasst, ausgewählt aus der Gruppe bestehend aus Niacin, Niacinamid, Zink-PCA, Zinkchlorid, Panthenol, Allantoin, Bisabolol, Biotin, Tocopherol, Tocopherylacetat und Taurin.

6. Verwendung nach einem der Ansprüche 1 bis 5, wobei die Zusammensetzung weiterhin mindestens einen zusätzlichen Bestandteil umfasst, ausgewählt aus der Gruppe, bestehend aus Haarkonditionierungsmitteln, Hautkonditionierungsmitteln, Feuchthaltemitteln, Konservierungsmitteln, Viskositätsvermittlern, Stabilisatoren, pH-Modifikatoren, Duftstoffen, Antioxidantien, Tensiden, Lichtschutzfiltern, Farbstoffen, Chelatbildnern und Lösungsmitteln.

7. Verwendung nach Anspruch 5 und/oder Anspruch 6, wobei jeder der zusätzlichen Wirkstoffe und/oder jeder der zusätzlichen Bestandteile in der Zusammensetzung in einer Menge von 0,001 bis 50 Gew.-%, bevorzugt in einer Menge von 0,001 bis 20 Gew.-%, stärker bevorzugt in einer Menge von 0,001 bis 10 Gew.-% vorhanden ist.

## Revendications

1. Utilisation cosmétique d'une composition comportant de la caféine pour empêcher et/ou traiter l'amincissement saisonnier des cheveux, dans laquelle la caféine est présente dans la composition en une quantité de 0,1 % à 10 % en poids, et dans laquelle l'amincissement saisonnier des cheveux est défini comme la réduction du diamètre des cheveux pendant la saison hivernale par rapport à la saison estivale.

2. Utilisation selon la revendication 1, dans laquelle la caféine est présente dans la composition en une quantité de 0,1 % à 5 % en poids.

3. Utilisation selon la revendication 1 et/ou la revendication 2, dans laquelle la composition est appliquée par voie topique.

4. Utilisation selon l'une quelconque des revendications 1 à 3, dans laquelle la composition est présente sous la forme d'une formulation cosmétiquement acceptable comportant un shampooing, une émulsion à laisser poser et à rincer et des revitalisants à base de gel, des cires et en plus des toniques à base d'éthanol ainsi que d'eau.

5. Utilisation selon l'une quelconque des revendications 1 à 4, dans laquelle la composition comporte en outre au moins un ingrédient actif supplémentaire choisi parmi le groupe constitué de niacine, niacinamide, PCA de zinc, chlorure de zinc, panthénol, allantoïne, bisabolol, biotine, tocophérol, acétate de tocophéryle et taurine.

6. Utilisation selon l'une quelconque des revendications 1 à 5, dans laquelle la composition comporte en outre au moins un composant supplémentaire choisi parmi le groupe constitué d'agents revitalisants pour les cheveux, d'agents revitalisants pour la peau, d'agents hydratants, de conservateurs, de viscosifiants, de stabilisants, de modificateurs de pH, de fragrances, d'anti-oxydants, de tensioactifs, de filtres de protection contre la lumière, de colorants, de chélateurs et de solvants.

7. Utilisation selon la revendication 5 et/ou la revendication 6, dans laquelle chacun des ingrédients actifs supplémentaires et/ou chacun des composants supplémentaires est présent dans la composition en une quantité de 0,001 % à 50 % en poids, de manière préférée en une quantité de 0,001 % à 20 % en poids, de manière plus préférée en une quantité de 0,001 % à 10 % en poids.
